# DEMANDE DE BREVET EUROPEEN

(11) **EP 0 781 545 A1**
(43) Date de publication de la demande: **02.07.1997**
(21) Numéro de dépôt: 96402674.4
(22) Date de dépôt: 09.12.1996
(51) Int. Cl.: A61K 7/48

(54) **Mélange comprenant des extraits végétaux pour l'hydratation des couches supérieures de l'épiderme**

(30) Priorité: 12.12.1995 FR 9514710
(71) Demandeur: LABORATOIRE DE BIOLOGIE VEGETALE YVES ROCHER, 56201 La Gacilly (FR)
(72) Inventeur: Prugnaud, Laurent, 94300 Vincennes (FR); Lubrano, Christian, 94240 L'Haye-Les-Roses (FR); Scott de Martinville, Anne-Marie, 80190 Nesle (FR)
(74) Mandataire: Le Guen, Gérard

(57) **Abrégé**

Cette invention a pour objet une émulsion de type huile dans l'eau pour l'hydratation des couches supérieures de l'épiderme, comprenant une phase lipidique dispersée dans une phase aqueuse,
- la phase lipidique étant composée de produits végétaux apolaires comprenant :
   - de 5 à 15 % en poids d'huiles végétales ;
   - de 6 à 15 % en poids de cires végétales ; et
- la phase aqueuse comprenant :
   - de 0,5 à 4 % en poids de gommes végétales;
   - de 2 à 7 % % en poids d'extraits végétaux riches en polysaccharides ;
   les pourcentages étant exprimés par rapport au poids total de l'émulsion.

## Description

La présente invention concerne un mélange comprenant des extraits végétaux pour l'hydratation des couches supérieures de l'épiderme.

L'eau fait partie de la constitution de la peau. Cette eau est distribuée de façon hétérogène. Il existe aussi un gradient décroissant du derme (qui contient 70 % d'eau) vers l'extérieur, l'épiderme ne contenant plus que 12 à 15 % d'eau.

L'eau de l'épiderme est pour l'essentiel intra cellulaire. Une peau dont le stratum corneum (SC) est bien hydraté, a un aspect agréable. C'est l'eau qui lui confère entre autre ses propriétés mécaniques et sa perméabilité. Le stratum corneum ne doit pas s'éloigner d'une valeur d'équilibre, sous peine d'une altération de ses propriétés mécaniques et physiologiques. Il est donc obligatoire de préserver l'hydratation de la peau.

Partant de l'étude du rôle de différents composés végétaux, notamment de végétaux de régions arides, dans la captation de l'eau et dans sa rétention au sein des tissus végétaux, les auteurs de la présente invention se sont intéressés aux moyens d'utiliser les propriétés hydratantes de ces composés pour le maintien d'une bonne hydratation de la peau.

Ceux-ci se sont plus particulièrement intéressés à deux types de composés à activité complémentaire et synergique, c'est-à-dire combinant un pouvoir hydratant et anti-déshydratant, pour améliorer durablement l'hydratation des couches supérieures de l'épiderme.

Les auteurs de la présente invention ont ainsi obtenu un mélange à partir de composés végétaux polaires (gommes végétales, polysaccharides) et de composés végétaux apolaires (cires et huiles végétales) capable d'être incorporé à titre de principe actif hydratant dans des compostions cosmétiques ou pharmaceutiques pour l'hydratation des couches supérieures de l'épiderme.

L'invention a donc pour objet une émulsion de type huile dans l'eau pour l'hydratation des couches supérieures de l'épiderme, comprenant une phase lipidique dispersée dans une phase aqueuse,
- la phase lipidique étant composée de produits végétaux apolaires comprenant :
   - de 5 à 15 % en poids d'huiles végétales ;
   - de 6 à 15 % en poids de cires végétales ; et
- la phase aqueuse comprenant :
   - de 0,5 à 4 % en poids de gommes végétales;
   - de 2 à 7 % % en poids d'extraits végétaux riches en polysaccharides ;
les pourcentages étant exprimés par rapport au poids total de l'émulsion.

Elle vise également des compositions cosmétiques ou pharmaceutiques pour l'hydratation des couches supérieures de l'épiderme comprenant cette émulsion à titre de principe actif hydratant.

Elle vise en outre l'utilisation de ladite émulsion pour la fabrication de compositions cosmétiques ou pharmaceutiques pour l'hydratation des couches supérieures de l'épiderme ainsi que l'utilisation de l'émulsion pour améliorer l'hydratation des couches supérieures de l'épiderme en cosmétologie.

L'invention concerne enfin une méthode pour améliorer l'hydratation des couches supérieures de l'épiderme par l'application d'une composition cosmétique comprenant l'émulsion sus-mentionnée à titre de principe actif.

L'émulsion selon l'invention comprend des constituants qui sont avantageusement sélectionnés en fonction de leurs propriétés hydratantes. En particulier, elle comprend :
- des gommes végétales et extraits végétaux riches en polysaccharides, choisis parmi un ou plusieurs des composés suivants : gomme de guar, gomme de caroube, gomme arabique, gomme adragante, gomme de sterculia, extrait de yucca, extrait d'aloe vera et extrait d'opuntia ;
- des huiles végétales, qui sont préférentiellement des huiles végétales riches en acides gras polyinsaturés (AGPI) choisies parmi un ou plusieurs des composés suivants : huile de balanites, huile de sésame et huile de graines de baobab ;
- des cires végétales choisies parmi la cire de carnauba, l'huile de jojoba (cire liquide) et la cire de candelilla.

Les extraits végétaux utilisés dans l'émulsion selon l'invention proviennent avantageusement de plantes capables de pousser dans des régions arides ou semi-arides.

Les polysaccharides végétaux de l'émulsion sont des mucilages qui possèdent la caractéristique de capter et de retenir fortement l'eau par leurs propriétés macromoléculaires hydrophiles.

Les huiles végétales limitent l'évaporation hydrique et agissent à deux niveaux : d'une part, grâce à leurs propriétés hydrophobes, d'autre part, grâce à l'incorporation des acides gras notamment insaturés dans le stratum corneum, qui vient renforcer l'efficacité de la barrière cutanée.

Enfin, les cires, présentes naturellement à la surface des feuilles, permettent de limiter les pertes hydriques des plantes grâce à leur nature hydrophobe. Incorporées dans l'émulsion selon l'invention, ces cires végétales permettent de limiter les pertes hydriques cutanées grâce à un effet filmogène et réducteur de la perte insensible en eau (PIE).

Selon une variante préférée, l'émulsion de l'invention comprend en outre 10 à 50 % en poids d'un polyol à haut pouvoir humectant choisi parmi, par exemple, la glycérine, le sorbitol, le saccharose ou le lactose.

Avantageusement, l'émulsion comprend également des molécules amphiphiles, telles en particulier les phospholipides, constituant naturel des membranes cellulaires. Ces phospholipides sont notamment choisis parmi les lécithines, (pouvant contenir différents pourcentages de phosphatidylcholine, phosphatidyléthanolamine ou de phosphatidylinositol) et sont préférentiellement présents à raison de 1 à 5 % en poids dans le mélange.

Un mélange sous forme d'émulsion préféré selon l'invention est composé de :

| | | | |
|---|---|---|---|
| Phosphatidylcholine | 1 à 3 % | et préférentiellement | 2 % en poids |
| Gomme de guar | 0,5 à 2 % | | 0,7 % " |
| Gomme de caroube | 0,5 à 2 % | | 0,7 % " |
| Aloe vera poudre | 0,5 à 2 % | | 1 % " |
| Yucca poudre | 2 à 5 % | | 2,5 % " |
| Glycérine | 15 à 40 % | | 40 % " |
| Huile de Balanites | 5 à 10 % | | 10 % " |
| Huile Jojoba | 5 à 10 % | | 10 % " |
| Cire de Carnauba | 1 à 5 % | | 3 % " |
| Eau | Q S | | |

Le taux d'incorporation d'une émulsion selon l'invention dans des compositions cosmétiques ou pharmaceutiques peut varier de 1,0 à 10 % selon le taux d'hydratation recherché.

La présentation sous forme d'émulsion permet une absorption de ses constituants par la peau plus facile, du fait d'un abaissement de la tension superficielle des corps gras, favorisant une intimité de contact beaucoup plus grande avec les éléments constitutifs de l'épiderme.

D'autre part, cette présentation permet de mélanger de façon intime des éléments de nature physique incompatible, mais de propriétés complémentaires. Celle-ci permet donc le cumul et la synergie d'activité de produits naturellement non miscibles.

L'émulsion selon l'invention présente de surcroît l'avantage de ne contenir que des produits et additifs naturels (sans modification chimique) ou des composés d'origine naturelle (composés naturels ayant subi des modifications chimiques).

Cette émulsion décrite ci-dessus peut être obtenue selon le protocole suivant :

Les extraits végétaux et gommes végétales sont des extraits secs sous forme de poudre, obtenus par extraction chimique ou à partir de sucs concentrés et séchés. Ces extraits végétaux et autres composés et additifs utilisés, sont disponibles auprès de fournisseurs. Les caractéristiques physico-chimiques de ces composés sont décrites dans la littérature ou communiquées par les fournisseurs.

Par exemple, un extrait d'aloe vera en poudre peut être obtenu par broyage et trituration de feuilles d'aloe vera pour fournir un gel liquide d'aloe vera, puis par lyophilisation de ce gel.

On peut également utiliser un produit à base de yucca en poudre contenant 50 % d'extrait de yucca disponible auprès de Garuda International. Ce produit est préparé par extraction à l'eau de tiges de yucca, ajout de maltodextrine pour obtenir une suspension dont l'extrait sec est constitué à 50 % en poids de maltodextrine et à 50 % de matière extraite du yucca, et séchage par atomisation de cette suspension pour fournir un produit final en poudre.

La préparation du mélange consiste à :
- incorporer dans un mélangeur de l'eau déminéralisée qui est chauffée entre 75 et 80°C ;
- dissoudre la fraction de phospholipides (lécithine) dans la phase aqueuse, sous forte agitation et chauffage pour qu'elle se disperse (le mélange devient alors laiteux et opalescent) ;
- prédisperser dans un récipient à part, les gommes et extraits végétaux dans la glycérine et vérifier l'homogénéité de ce prémélange ;
- introduire ce prémélange dans la phase aqueuse sous forte agitation puis laisser gonfler les gommes végétales pendant 30 minutes à une température comprise entre 70 et 80°C ;
- en parallèle, chauffer les cires et les huiles à 80°C et vérifier la bonne fusion des différents constituants ;
- après avoir vérifié l'homogénéité des deux phases, introduire la phase grasse sur la phase aqueuse puis agiter 5 à 10 minutes à grande vitesse ;
- refroidir sous agitation pour ramener le mélange à 25°C.

Il est alors obtenu un mélange de type émulsion huile dans l'eau qui sera ultérieurement utilisé pour la fabrication de compositions cosmétiques ou phrmaceutiques pour l'hydratation des couches supérieures de l'épiderme.

A titre d'illustration, on décrira ci-après les propriétés hydratantes de l'émulsion (mélange I) comprenant les composés suivants :

| | |
|---|---|
| Phosphatidylcholine | 2 % en poids |
| Gomme de guar | 0,7 % " |
| Gomme de caroube | 0,7 % " |
| Aloe vera poudre | 1 % " |
| Yucca poudre | 2,5 % " |
| Glycérine | 40 % " |
| Huile de Balanites | 10 % " |
| Huile Jojoba | 10 % " |
| Cire de Carnauba | 3 % " |
| Eau | QS |

L'activité hydratante de l'émulsion est mesurée par cornéométrie sur l'avant-bras. Son efficacité, lorsque celui-ci est incorporé à raison de 2% dans une composition cosmétique, est ensuite mesurée par conductivité thermique.

La méthode par cornéométrie consiste à mesurer les propriétés diélectriques de la peau avant et après application du produit sur une surface définie.

Les résultats exprimés en unités arbitraires sont transformés en pourcentage d'augmentation.

La méthode par conductivité thermique permet de mesurer les variations thermiques cutanées, qui sont liées aux variations de la teneur en eau de la peau. Les résultats sont exprimés en Cal/cm x °C x S et peuvent être exprimées en % d'augmentation.

Les résultats des mesures par cornéométrie et par conductivité thermiques sont représentés sur les figures ci-après dans lesquelles :

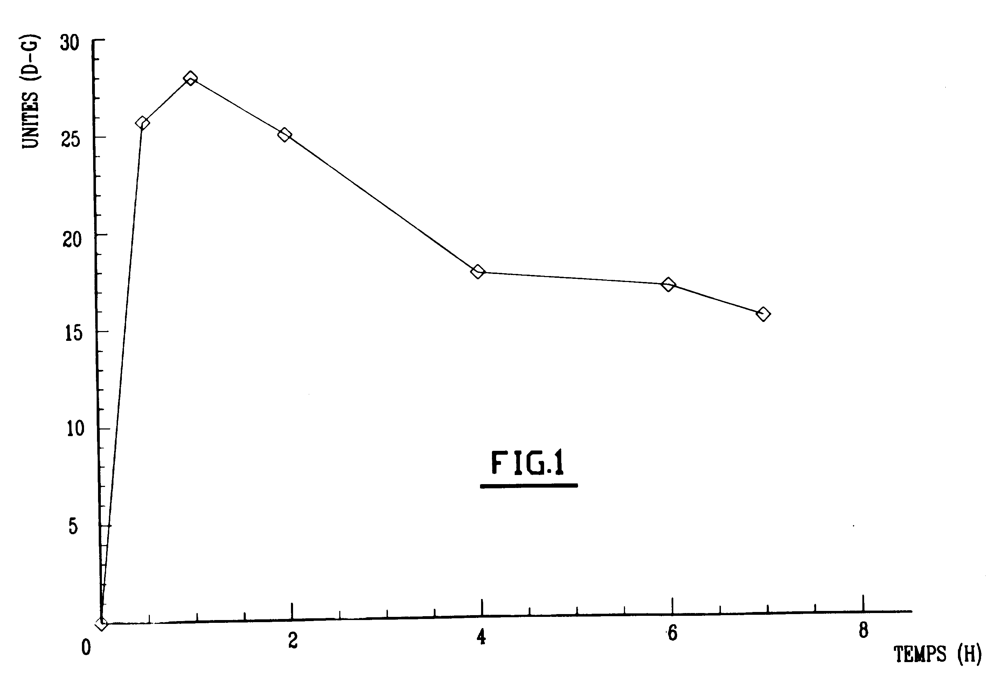

- la figure 1 représente la mesure par cornéométrie du taux d'hydratation du mélange I appliqué sur un avant-bras pendant 7 heures (unités arbitraires);

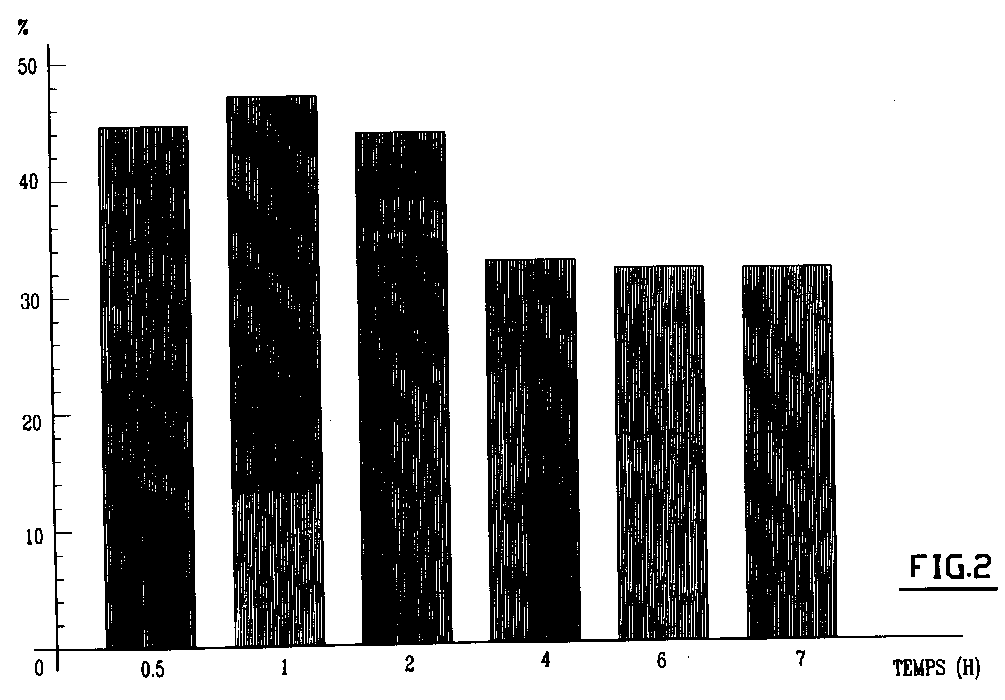

- la figure 2 représente l'augmentation du taux d'hydratation sur le même mélange exprimé en % ;

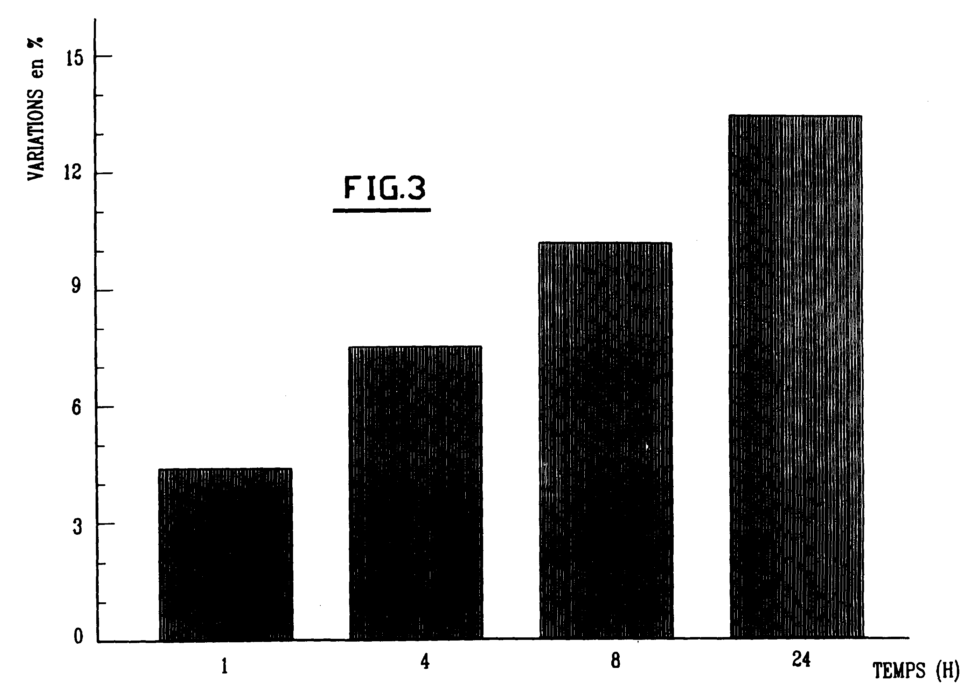

- la figure 3 représente la mesure de l'effet hydratant d'une composition cosmétique comprenant 2 % du mélange I selon l'invention (par conductivimètre, 12 cas sur 24).

Les mesures par cornéométrie indiquent que le mélange de l'invention permet d'augmenter le taux d'hydratation de la peau de plus de 40 %, deux heures après son application.

L'hydratation se maintient ensuite à un niveau d'environ 30 % jusqu'à 7 heures après son application. Par ailleurs, les mesures par conductivité thermique permettent de vérifier que l'activité hydratante du mélange incorporé dans une composition cosmétique se manifeste jusqu'à 24 heures où l'on peut observer un pourcentage d'augmentation d'environ 13 %.

Les exemples suivants de compositions sous différentes formes illustrent la présente invention.

Le mélange hydratant utilisé dans ces exemples de compositions est le mélange I défini précédemment.

### EXEMPLES

### EXEMPLE 1 : Composition sous forme de crème

### Phase A

| | |
|---|---|
| Eau déminéralisée | qsp 100 % en poids |
| Conservateurs | qsp " |
| Mélange hydratant (mélange I) | 4,00 % " |
| Allantoine | 0,10 % " |
| Tetrasodium EDTA | 0,05 % " |
| Carbomer | 0,20 % " |

### Phase B

| | |
|---|---|
| Triglycérides C8/C10 | 3,00 % " |
| α bisabolol | 0,20 % " |
| Huile végétale | 7,00 % " |
| Cyclométhicone | 4,00 % " |
| Méthoxy PEG 17/dodecyl glycol Copolymère | 2,00 % " |
| Beurre de karité | 5,00 % " |

### Phase C

| | |
|---|---|
| L lysine | qsp pH 5.8-6.3 |
| Parfum | qs |

### EXEMPLE 2 : Composition sous forme de lait

### Phase A

| | |
|---|---|
| Eau déminéralisée | qsp 100 % en poids |
| Conservateurs | qsp " |
| Mélange hydratant (mélange I) | 3,00 % " |
| Tetrasodium EDTA | 0,05 % " |
| Carbomer | 0,10 % " |

### Phase B

| | |
|---|---|
| Cocoate éthyl hexyl | 7,00 % " |
| Adipate d'isopropyl | 5,00 % " |
| Cetyl palmitate | 1,00 % " |
| Huile végétale | 3,00 % " |
| Diméthicone | 2,00 % " |
| Alcool gras éthoxylé | 3,00 % " |

### Phase C

| | |
|---|---|
| NaOH | qsp pH 6,00 |
| Parfum | qs |

### EXEMPLE 3 : Composition sous forme de lotion

### Phase A

| | |
|---|---|
| Eau déminéralisée | qsp 100 % en poids |
| Tetrasodium EDTA | 0,05 % " |
| PEG 400 | 2,00 % " |
| Conservateurs | qs |
| | |
| Alcool 96 ° | 5,00 % " |
| Alcool oléique 20 OE | 0,50 % " |
| Parfum | qsp |
| | |
| Mélange hydratant (mélange I) | 1,50 % " |
| | |
| Colorant | qs |

### EXEMPLE 4 : Composition sous forme de gel

### Phase A

| | |
|---|---|
| Eau déminéralisée | qsp 100 % en poids |
| Conservateurs | qs |
| Carbomer | 0,60 % " |
| Gomme de xanthane | 0,10 % " |
| Mélange hydratant (mélange I) | 2,00 % " |

### Phase B

| | |
|---|---|
| Ethanol 96° | 10,00 % " |
| Diméthicone copolyol | 3,00 % " |
| Parfum | qs |

### Phase C

| | |
|---|---|
| NaOH | qsp pH 6,00 |
| Colorants | qs |

## Revendications

1. Emulsion de type huile dans l'eau pour l'hydratation des couches supérieures de l'épiderme, comprenant une phase lipidique dispersée dans une phase aqueuse,
- la phase lipidique étant composée de produits végétaux apolaires comprenant :
- de 5 à 15 % en poids d'huiles végétales ;
- de 6 à 15 % en poids de cires végétales ; et
- la phase aqueuse comprenant :
- de 0,5 à 4 % en poids de gommes végétales;
- de 2 à 7 % % en poids d'extraits végétaux riches en polysaccharides ;
les pourcentages étant exprimés par rapport au poids total de l'émulsion.

2. Emulsion selon la revendication 1, caractérisée en ce que les gommes végétales et extraits végétaux riches en polysaccharides sont choisis parmi un ou plusieurs des composés suivants : gomme de guar, gomme de caroube, gomme arabique, gomme adragante, gomme de sterculia, extrait de yucca, extrait d'aloe vera et extrait d'opuntia.

3. Emulsion selon la revendication 1, caractérisée en ce que les huiles végétales sont des huiles végétales riches en acides gras polyinsaturés choisies parmi un ou plusieurs des composés suivants : huile de balanites, huile de sésame et huile de graines de baobab.

4. Emulsion selon la revendication 1, caractérisée en ce que les cires végétales sont choisies parmi la cire de carnauba, l'huile de jojoba et la cire de candelilla.

5. Emulsion selon l'une quelconque des revendications précédentes, caractérisée en ce qu'elle comprend en outre 10 à 50 % d'un polyol choisi parmi la glycérine, le sorbitol, le saccharose ou le lactose et de 1 à 5 % de phospholipides.

6. Emulsion selon l'une quelconque des revendications précédentes comprenant les composés suivants :
| | |
|---|---|
| Phosphatidylcholine | 1-3 % en poids |
| Gomme de guar | 0,5-2 % " |
| Gomme de caroube | 0,5-2 % " |
| Aloe vera poudre | 0,5-2 % " |
| Yucca poudre | 2-5 % " |
| Glycérine | 15 à 40 % " |
| Huile de Balanites | 5 à 10 % " |
| Huile Jojoba | 5 à 10 % " |
| Cire de Carnauba | 1 à 5 % " |
| Eau | QS |

7. Composition cosmétique ou pharmaceutique pour l'hydratation des couches supérieures de l'épiderme, caractérisée en ce qu'elle comprend de 1 à 10 % d'une émulsion hydratante selon l'une quelconque des revendications 1 à 6 à titre de principe actif.

8. Composition selon la revendication 7, caractérisée en ce qu'elle se présente sous forme de lait, lotion, crème ou gel.

9. Méthode pour améliorer l'hydratation des couches supérieures de l'épiderme qui comprend l'application d'une composition cosmétique selon la revendication 7 ou 8.

10. Utilisation d'une émulsion selon l'une quelconque des revendications 1 à 6 pour la fabrication d'une composition cosmétique ou pharmaceutique pour l'hydratation des couches supérieures de l'épiderme.

11. Utilisation d'une émulsion selon l'une quelconque des revendications 1 à 6, pour améliorer l'hydratation des couches supérieures de l'épiderme en cosmétologie.
